# EUROPEAN PATENT APPLICATION

(11) **EP 2 447 366 A1**
(43) Date of publication of application: **02.05.2012**
(21) Application number: 11187142.2
(22) Date of filing: 28.10.2011
(51) Int. Cl.: C12N 15/10

(54) **Method of synthesizing single-stranded cDNA, method of preparing microarray sample, and method of detecting nucleic acid**

(30) Priority: 29.10.2010 JP 2010244102
(71) Applicant: Sysmex Corporation, Kobe-shi, Hyogo 651-0073 (JP)
(72) Inventor: Yoshida, Yuichiro, Kobe-shi, Hyogo 651-0073 (JP)
(74) Representative: Paemen, Liesbet R.J.

(57) **Abstract**

In order to provide a method of amplifying nucleic acid which can produce a nearly constant amount of amplified products irrespective of the amount of RNA to be used as a template, the primer is used at a final concentration of 500 pM to 500 nM in a reaction which synthesizes a single-stranded cDNA using RNA extracted from a biological sample as a template and using a primer with oligo dT and a promoter sequence.

## Description

### FIELD OF THE INVENTION

The present invention relates to a method of synthesizing a single-stranded cDNA and a method of preparing a sample to be subjected to detection of nucleic acid using a microarray from the single-stranded cDNA obtained by the synthesizing method. Further, the present invention relates to a method of detecting a target nucleic acid in a sample with a microarray.

### BACKGROUND

Recently, microarrays have gained interest as tools for detecting a target nucleic acid. A microarray, which is also referred to as "a DNA chip" includes a substrate such as a plastic or glass substrate and nucleic acid fragments that are placed as probes at high density on the substrate. A large number of genes in a sample can be analyzed simultaneously and comprehensively by hybridizing the probes on the microarray with nucleic acids (DNA, RNA, cDNA, or cRNA) in the sample. Thus, microarrays are extremely useful for the analysis of gene expression and variation. Attempts to apply the microarray to clinical tests have also started.

In the detection of a nucleic acid using a microarray, the detection accuracy of nucleic acid is reduced when there is only a small amount of the target nucleic acid in the sample . Therefore, in order to improve the detection accuracy of nucleic acids with a microarray, methods for amplifying a nucleic acid in a sample to prepare a microarray sample have been developed (PCT International Publication No. WO 01/038572), U.S. Patent Publication No. 2002/127592, and Japanese Patent Application Laid-Open (JP-A) No. 2007-300829).

Conventionally, when gene expression is analyzed with a microarray, a single-stranded cDNA is synthesized by extracting RNA from a biological sample and using it as a template. A double-stranded cDNA is synthesized from the single-stranded cDNA and cRNA is amplified from the double-stranded cDNA by an in vitro transcription (IVT) reaction to obtain a microarray sample. The concentration of cRNA included in the microarray sample is in many cases too high to be subjected to the microarray. Therefore, the cRNA is quantified and diluted to a suitable concentration before subjecting the sample to the microarray.

Thus, when the target nucleic acid is detected with the microarray using a sample obtained by the conventional nucleic acid amplification, it is necessary to perform one or more complicated operations such as the quantification of the nucleic acid concentration included in the sample and dilution of the sample.

The detection of nucleic acid with the microarray generally includes the following processes (1) to (4): (1) extracting nucleic acid from a biological sample; (2) amplifying the extracted nucleic acid; (3) hybridizing the amplified nucleic acid product with probes on the microarray; and (4) measuring the microarray. Currently, an apparatus which automates the processes of (3) and (4) is commercially available. However, an apparatus which automates the whole processes has not yet been developed. This is because a judgment of measuring the concentration of nucleic acid in the sample and appropriately diluting it according to the measurement results is needed in the process from (2) to (3) and it is difficult to automate the concentration measurement and dilution operations.

### SUMMARY OF THE INVENTION

The scope of the present invention is defined solely by the appended claims, and is not affected to any degree by the statements within this summary.

An objective of the present invention is to provide a method of synthesizing a single-stranded cDNA which does not require quantification and dilution and is used for preparing a microarray sample. Another objective of the present invention is to provide a method of preparing a microarray sample using the method of synthesizing a single-stranded cDNA and a method of detecting a nucleic acid with a microarray.

The present inventors have found that a nearly constant amount of cRNA can be obtained by using a primer at a very low concentration to obtain a single-stranded cDNA and synthesizing cRNA from the single-stranded cDNA in the synthetic reaction of the single-stranded cDNA irrespective of the amount of RNA used as a template.
(1) A first aspect of the present invention is a method of synthesizing a single-stranded cDNA, comprising:
   synthesizing a single-stranded cDNA using RNA extracted from the biological sample as a template and using a primer with oligo dT and a promoter sequence;
   wherein the primer is used at a final concentration of 500 pM to 500 nM in the synthesis of the single-stranded cDNA. In particular embodiments the primer is used at a final concentration of 500nM or less, 200 nM or less, 100 nM or less, 50 nM or less, 10 nM or less, or 5nM or less.

According to the method of synthesizing a single-stranded cDNA of the present invention, irrespective of the amount of RNA used as a template, a nearly constant amount of single-stranded cDNA can be obtained. Therefore, quantification and dilution processes after extraction of RNA from the biological sample can be omitted.
(2) The synthesizing method according to (1), wherein the biological sample is blood, lymph, urine, tissue, or cell samples.
(3) The synthesizing method according to (1) or (2), wherein
   the concentration of RNA to be used as a template which is included in a reaction solution in the reaction for synthesizing a single-stranded cDNA is from 0.005 to 0.5 µg/µL.
(4) A second aspect of the present invention is method of preparing a microarray sample, comprising:
   extracting RNA from a biological sample;
   synthesizing a single-stranded cDNA using a reaction solution containing the extracted RNA and a primer with oligo dT and a promoter sequence; and
   synthesizing a target nucleic acid using the synthesized cDNA as a template to prepare a microarray sample;
   wherein the final concentration of the primer in the reaction solution in the process of synthesizing a single-stranded cDNA is from 500 pM to 500 nM. In particular embodiments the primer is used at a final concentration of 500nM or less, 200 nM or less, 100 nM or less, 50 nM or less, 10 nM or less, or 5nM or less. The present invention further provides the use of the methods of preparing a microarray according to the present invention using biological samples comprising unknown or varying amounts of template RNA. In the method of preparing a microarray sample of the present invention, the single-stranded cDNA obtained by the synthesizing method is used, and thus a target nucleic acid in the obtained sample is present in an almost constant amount. Therefore, in the method of preparing a microarray sample of the present invention, quantification and dilution processes of nucleic acid after preparation of the sample can be omitted.
(5) The method of preparing a microarray sample according to (4), wherein
   the biological sample is blood, lymph, urine, tissue, or cell samples.
(6) The method of preparing a microarray sample according to (4) or (5), wherein
   the concentration of RNA to be used as a template in the process of synthesizing a single-stranded cDNA is from 0.005 to 0.5 µg/µL.
(7) The method of preparing according to any one of (4) to (6), wherein
   the target nucleic acid in the process of preparing a microarray sample is cRNA.
(8) A third aspect of the present invention is a method of detecting a nucleic acid with a microarray, comprising:
   extracting RNA from a biological sample;
   synthesizing a single-stranded cDNA using a reaction solution containing the extracted RNA and a primer with oligo dT and a promoter sequence;
   synthesizing a target nucleic acid using the synthesized cDNA as a template to prepare a microarray sample;
   bringing the prepared microarray sample into contact with a microarray and hybridizing the target nucleic acid included in the microarray sample with probes arranged on the microarray;
   measuring a signal generated by hybridization of the target nucleic acid and the probe; and
   detecting the target nucleic acid from the microarray sample based on the measurement results;
   wherein the final concentration of the primer in a reaction solution for synthesizing a single-stranded cDNA in the process of synthesizing a single-stranded cDNA is from 500 pM to 500 nM. In particular embodiments the primer is used at a final concentration of 500nM or less, 200 nM or less, 100 nM or less, 50 nM or less, 10 nM or less, or 5nM or less.

The present invention further provides the use of the methods of detecting of the present invention for the analysis of samples comprising unknown or varying amounts of template RNA.

The method of detecting nucleic acid of the present invention which detects the target nucleic acid included in the sample with the microarray does not need the steps of quantification and dilution of nucleic acid, so that it can be suitably used for a system to automate a process from the preparation of the microarray sample to the microarray measurement.
(9) The method of detecting a nucleic acid with a microarray according to (8), wherein
   the biological sample is blood, lymph, urine, tissue, or cell samples.
(10) The method of detecting a nucleic acid with a microarray according to (8) or (9), wherein
   the concentration of RNA to be used as a template in the process of synthesizing a single-stranded cDNA is from 0.005 to 0.5 µg/µL.
(11) The method of detecting a nucleic acid with a microarray according to any one of (8) to (10), wherein
   the target nucleic acid in the process of preparing a microarray sample is cRNA.

The present invention further provides the use of the methods of the present invention to obtain a predetermined amount of cRNA or cDNA. More particularly the use of the methods of the present invention to obtain a predetermined amount of cRNA or cDNA starting from unknown or varying amounts of template RNA.

### BRIEF DESCRIPTION OF THE DRAWINGS

Fig. 1 is a graph showing the relationship between a final concentration of T7-oligo dT primer and a cRNA yield;
Fig. 2 is a graph showing the relationship between the amount of RNA used as a template and the cRNA yield;
Fig. 3 is a graph showing correlation when cRNA synthesized from different amounts of RNA is detected with a microarray;
Fig. 4 is a graph showing the relationship between the final concentration of NTP mix and the cRNA yield; and
Fig. 5 is a graph showing the relationship between IVT reaction time and the cRNA yield in a reaction system using different concentrations of NTP mix.

### DETAILED DESCRIPTION OF THE PREFERRED EMBODIMENTS

The preferred embodiments of the present invention will be described hereinafter with reference to the drawings.

### [1. Method of synthesizing single-stranded cDNA]

Hereinafter, the method of synthesizing a single-stranded cDNA according to the embodiment (hereinafter simply referred to as "the synthesizing method") will be described. In the synthesizing method of the present invention, RNA extracted from a biological sample is used as a template. The biological sample is not particularly limited as long as it is a sample derived from a living body from which RNA can be extracted. Examples thereof include blood extracted from the living body (whole blood, blood plasma, and serum are included), lymph, urine, cell, and tissue samples. The RNA to be used as a template in the synthesizing method of the present invention may be an RNA fraction which can be extracted from the biological sample by any known method in the art and it is preferably mRNA. mRNA in eukaryotic organisms has a polyadenylic acid (poly A) sequence called a poly (A) tail at its 3'end.

The method of extracting RNA from a biological sample is not particularly limited. The method can be performed by adding denaturants such as phenol and guanidine thiocyanate to the biological sample and releasing mRNA from the sample to a solution. If necessary, RNA extracted from the biological sample may be purified. Examples of the method of purifying RNA include a phenol/chloroform method, a density gradient centrifugation method, and an oligo dT column method. In the purification and extraction of RNA, commercially available RNA extraction/purification kits can also be used. When the biological sample is blood, GLOBINclear-Human kit (Ambion) may be used in order to remove mRNA of globin. In the synthetic reaction of the present invention, the amount of RNA to be used as a template is not particularly limited. To give one example thereof, the amount is usually from 0.1 to 10 µg, preferably from 1.0 to 10 µg in the case of 20 µl of reaction system. That is, the RNA concentration is from 0.005 to 0.5 µg/µL, preferably from 0.05 to 0.5 µg/µL.

The primer to be used for the synthesizing method of the present invention has a promoter sequence at the 5'end side and has an oligo dT sequence at the 3'end side. The number of T of the oligo dT sequence is not particularly limited as long as the primer is annealed to a poly A sequence of mRNA via the oligo dT sequence to synthesize the single-stranded cDNA. The number is usually from 15 to 40, preferably 40. The promoter sequence is not particularly limited as long as it is a sequence capable of recognizing RNA polymerase and performing the IVT reaction. Examples thereof include T7, T3, and Sp6 promoters. The primer to be used for the synthesizing method of the present invention may include other sequences as long as it does not inhibit the synthetic reaction of cRNA from a double-stranded cDNA using the promoter sequence in the method of preparing a sample of the present invention to be described later. The primer itself can be synthesized by any known method in the art. A commercially available primer with oligo dT and a promoter sequence may be used.

In the synthesizing method of the present invention, the primer with oligo dT and a promoter sequence is used at a final concentration of 500 pM to 500 nM in a reaction solution as described above. In particular embodiments the primer is used at a final concentration of 500nM or less, 200 nM or less, 100 nM or less, 50 nM or less, 10 nM or less, or 5nM or less. The primer concentration is much lower than the general concentration in the art (usually 5 µM). When the primer is used at a very low concentration, the primer is depleted during the reverse transcription reaction and the synthesis of a single-stranded cDNA is terminated. As a result, even if RNA used as a template is any amount, a nearly constant amount of single-stranded cDNA can be obtained. The term "nearly constant amount of single-stranded cDNA" includes that the obtained single-stranded cDNA has an amount (concentration) which can be used for the following synthetic reaction of double-stranded cDNA without diluting all or part of the single-stranded cDNA. In particular embodiments the term "nearly constant amount" refers to the a variation of less than 50%, more particularly less than 30%, even more particularly less than 20%, most particularly less than 10% or less than 15%, when different samples are compared, more particularly when starting from samples comprising an unknown amount of template RNA, more particularly samples comprising varying amounts of template RNA.

In the synthetic reaction of the present invention, any known reverse transcriptase in the art is used. Examples of the reverse transcriptase include Avian Myeloblastosis Virus (AMV) reverse transcriptase and Molony Murine Leukemia Virus (M-MLV) reverse transcriptase.The synthetic reaction of the present invention can be performed in the same manner as the reverse transcription reaction of RNA commonly used in the art except that the final concentration of the primer is set to 500 pM to 500 nM. If necessary, the synthesized single-stranded cDNA may be purified. Any method known in the art such as ethanol precipitation as well as commercially available nucleic acid purification kits can be used for the purification.

### [2. Method of preparing sample]

The method of preparing a microarray sample to be subjected to detection of nucleic acid using a microarray according to this embodiment (hereinafter simply referred to as "the preparing method") will be described below.The preparing method of the present invention is performed by synthesizing a target nucleic acid using a single-stranded cDNA which can be obtained by the synthesizing method as a template. The target nucleic acid can be suitably selected depending on the microarray to be used. Usually, cDNA, cRNA, and the like are used. Any known method in the art can be used in the process of synthesizing a target nucleic acid. Here, as an example of the preparing method, the case where the target nucleic acid is cRNA will be described. First, a double-stranded cDNA is synthesized by using a single-stranded cDNA which can be obtained by the synthesizing method as a template and using a DNA polymerase. Since the single-stranded cDNA obtained by the synthesizing method of the present invention has a nearly constant amount, it is not necessary to quantify and dilute the single-stranded cDNA before the process of synthesizing the double-stranded cDNA. Any known method in the art, such as a nick translation method can be used in the process of synthesizing the double-stranded cDNA. In the nick translation method, a double-stranded cDNA can be synthesized by, for example, inserting a nick to a template RNA which is hybridized with a single-stranded cDNA by RNase H and replacing the template RNA to a DNA strand with DNA ligase derived from Escherichia coli (E. coli) and DNA polymerase I. Commercially available cDNA synthesis kits can also be used. If necessary, the synthesized double-stranded cDNA may be purified. Any method known in the art such as ethanol precipitation as well as commercially available nucleic acid purification kits can be used for the purification.

Subsequently, a microarray sample is obtained by synthesizing cRNA using the double-stranded cDNA thus obtained as a template and using a RNA polymerase corresponding to the promoter sequence of the primer. The sample thus obtained includes a nearly constant amount of cRNA. The term "nearly constant amount of cRNA" used herein includes that the sample containing cRNA has an amount (concentration) which can be used for detection of nucleic acid with a microarray without diluting all or part of the sample. In particular embodiments the term "nearly constant amount" refers to the a variation of less than 50%, more particularly less than 30%, even more particularly less than 20%, most particularly less than 10% or less than 15%, when different samples are compared, more particularly when starting from samples comprising an unknown amount of template RNA, more particularly samples comprising varying amounts of template RNA. Here, the RNA polymerase corresponding to the promoter sequence is an enzyme which can recognize the promoter sequence of the primer to perform the IVT reaction. For example, when the promoter sequence of the primer is a T7 promoter, a T7 RNA polymerase is used. The synthetic reaction of cRNA from the double-stranded cDNA in the process can be performed by any known method in the art. Commercially available cRNA synthesis kits can also be used. If necessary, the synthesized cRNA may be purified. Any method known in the art such as ethanol precipitation as well as commercially available nucleic acid purification kits can be used for the purification.

Since the microarray sample obtained by the preparing method of the present invention is subjected to microarray measurement, it is preferable to fragment cRNA in order to facilitate the hybridization of the cRNA in a sample with a probe. The fragmentation of cRNA can be performed by any known method in the art. Examples thereof include a method of heating in the presence of metal ions and a method of using enzymes such as ribonuclease.

In the method of detecting a nucleic acid with a microarray to be described later, when hybridization of cRNA in the microarray sample with the probe is measured by detecting a fluorescent substance or pigment, it is preferable that the cRNA is labeled with a labeling substance. Therefore, it is preferable that the preparing method of the present invention further includes a process of labeling the synthesized cRNA. The labeling substance is not particularly limited as long as it is a substance usually used in the art. Examples thereof include fluorescent substances such as Cy3, Cy5, Alexa Fluor (trademark), fluorescein isothiocyanate (FITC); haptens such as biotin; and radioactive substances. Methods of labeling cRNA with a labeling substance are known in the art. For example, in the process of synthesizing cRNA, biotin-labeled cRNA can be obtained by mixing biotinylated-ribonucleotide as a substrate with a reaction solution.

### [3. Method of detecting nucleic acid using microarray]

The method of detecting a nucleic acid with a microarray according to this embodiment (hereinafter simply referred to as "the detecting method") will be described below.

In the detecting method of the present invention, a sample which can be obtained by the method of preparing a microarray sample is first brought into contact with a microarray and then a target nucleic acid in the sample is hybridized with probes arranged on the microarray.

Here, the term "hybridization" means that the probe is hybridized with cRNA in the microarray sample under stringent conditions, for example, when the target nucleic acid is cRNA. The stringent conditions are not particularly limited as long as they are conditions which are generally used by those skilled in the art when hybridizing probes arranged on a microarray with cRNA in a sample.

The microarray which can be used for the detecting method of the present invention is not particularly limited as long as the nucleic acid probes are arranged on the substrate. A DNA microarray (DNA chip) is preferred. Such a microarray can be obtained by purchasing a commercially available microarray such as GeneChip (registered trademark) (Affymetrix) or can be produced in accordance with any known method in the art.

The contact of the microarray sample with the microarray can be performed by directly adding the sample obtained by the preparing method of the present invention. That is, in the detecting method of the present invention, it is unnecessary to quantify nucleic acid in the microarray sample in order to dilute to a concentration suitable for the microarray measurement before the contact. The contact may be performed at about 10 to 70°C for 2 to 20 hours, however the conditions vary depending on the type of microarray. Further, the contact can be performed by using Hybridization Oven 640, manufactured by Affymetrix.

The microarray may, after the contact with the microarray sample, be subjected to staining and cleaning processes. When the measurement of the signal in the next process is based on fluorescence intensity or coloring intensity, the hybrid of the target nucleic acid with the probe can be detected by the staining process. For example, when the target nucleic acid in the sample is labeled with biotin, avidin or streptavidin labeled with a suitable fluorescent substance in advance can be bound to the biotin-labeled hybrid on the microarray. This allows the hybrid to be stained. Examples of the fluorescent substance include FITC, Alexa Fluor (trademark), green fluorescent protein (GFP), luciferin, and phycoerythrin. A conjugate of phycoerythrin-streptoavidin is commercially available, and thus the use of the conjugate is convenient.

The hybrid can also be stained by binding avidin or streptavidin to biotin and bringing a labeled antibody to avidin or streptoavidin into contact with the microarray.

The staining and cleaning of the microarray can be performed by using a device such as Fluidic Station 450, manufactured by Affymetrix.

In the next step of the detecting method of the present invention, a signal generated by hybridization of the target nucleic acid in the sample and the probe is measured. Although the signal varies depending on the type of microarray, it may be, for example, an electrical signal generated by hybridization of the target nucleic acid and the probe (change in the amount of current). When the target nucleic acid is labeled as described above, the signal may be signals (fluorescence intensity, coloring intensity, etc.) such as fluorescence and coloring generated from the labeling substance. Among those signals, a signal generated from the labeling substance is preferred and a fluorescent signal is more preferred. Measurement of the signals can be performed by using a scanner included in a usual microarray measuring device. Examples of the scanner include GeneChip (registered trademark) Scanner 3000 7G (Affymetrix).

In the detecting method of the present invention, a target nucleic acid is detected from the microarray sample based on the signal measurement results. The term "detecting" used herein means a process of detecting the presence or absence of a target nucleic acid in a microarray sample. When the target nucleic acid is present in the microarray sample, a process of measuring the amount of nucleic acid in the sample is included in the scope of the detecting method of the present invention. The detection of the target nucleic acid from the microarray sample based on the results of the signal measurement can be performed by any known method in the art. The presence or absence of the target nucleic acid in the microarray sample or the abundance thereof can be detected by analyzing signal measured signal values obtained from the microarray using softwares such as GeneChip (registered trademark) Operating Software (Affymetrix) and ArrayAssist (Stratagene).

Hereinafter, the present invention will be described in detail with reference to examples, however the present invention is not limited thereto.

### [Examples]

### Test example 1: Examination of final concentration of primer in synthetic reaction of single stranded cDNA

### (1) Extraction of RNA from biological sample

Total RNA were extracted from K562 cells using an RNeasy mini kit (QIAGEN) according to the following procedures. All reagents and columns were included in the RNeasy mini kit.

693 µl of RLT buffer and 7 µl of 2-mercaptoethanol were added to the K562 cells (1 x 10⁶ cells), which was mixed. Further, 500 µl of 100% ethanol was added to the resultant mixture, which was mixed by pipetting. The mixture was applied to a column and centrifuged at 10,000 x g and 25°C for 15 seconds and then the filtrate was discarded. 350 µl of RW1 was applied to the column and centrifuged at 10,000 x g and 25°C for 15 seconds and then the filtrate was discarded. A mixture of 10 µl of DNase solution and 70 µl of RDD buffer was applied to the column and allowed to stand at room temperature for 20 minutes. 350 µl of RW1 was applied to the column and centrifuged at 10,000 x g and 25°C for 15 seconds and then the filtrate was discarded. 500 µl of RPE buffer was applied to the column and centrifuged at 10,000 x g and 25°C for 15 seconds and then the filtrate was discarded. Further, 500 µl of RPE buffer was applied to the column and centrifuged at 10,000 x g and 25°C for 5 minutes, followed by transferring of the column to a new collection tube. 30 µl of RNase-free water was applied to the center of the column and allowed to stand at room temperature for 1 minute, followed by centrifugation at 14,000 rpm and 25°C for 1 minute. Further, 30 µl of RNase-free water was applied to the center of the column and allowed to stand at room temperature for 1 minute, followed by centrifugation at 14,000 rpm and 25°C for 1 minute to obtain a total RNA solution.

### (2) Preparation of biotin-labeled cRNA

The obtained total RNA was used as a template and a single-stranded cDNA was synthesized using T7-oligo dT primer at final concentrations of 5pM, 500 pM, 5nM, 50nM, 500 nM, and 5 µM. Biotin-labeled cRNA was prepared from the single-stranded cDNA. Specifically, the biotin-labeled cRNA was prepared using GeneChip One-Cycle Target Labeling and Control Reagents (manufactured by Affymetrix) according to the following procedures.

### (2-1) Synthesis of single-stranded cDNA

The following reagents were charged to a PCR tube, which was incubated at 70°C for 10 minutes, subsequently at 4°C for 2 minutes. The sequence of T7-oligo dT primer is as shown in SEQ ID NO: 1.

| |
|---|
| Total RNA (1 µg) 3 µl |
| RNase-free water 5 µl |
| 20-fold diluted Poly-A RNA Control 2 µl |
| T7-oligo dT primer 2 µl |
| Total 12 µl |

The following reagents were added thereto, which was tapped.

| |
|---|
| 5 x First Strand Reaction Mix 4 µl |
| DTT 0.1 M 2 µl |
| dNTP 10 mM 1 µl |
| Total 7 µl |

The resultant product was incubated at 42°C for 2 minutes and 1 µl of Super Script II was added thereto, which was incubated at 42°C for 1 hour, subsequently at 4°C for 2 minutes or more to synthesize a single-stranded cDNA.

### (2-2) Synthesis of double-stranded cDNA

The following reagents were added to the synthesized single-stranded cDNA, which was tapped.

| |
|---|
| RNase-free water 91 µl |
| 5 × 2^{nd} Strand Reaction Mix 30µl |
| dNTP 10mM 3 µl |
| E. coli DNA ligase 1 µl |
| E. coli DNA polymerase I 4 µl |
| RNaseH 1 µl |
| Total 130 µl |

The mixture was incubated at 16°C for 2 hours. 2 µl of T4 DNA-polymerase I was added thereto, which was incubated at 16°C for 5 minutes. 10 µl of 0.5M EDTA was added thereto and a double-stranded cDNA was synthesized.

### (2-3) Cleaning of cDNA

The synthesized double-stranded cDNA was transferred to a 1.5-mL tube and 600 µl of cDNA Binding Buffer was added thereto, which was mixed with the vortex. 500 µl of the mixture was placed in a cDNA Cleanup Spin Column and centrifuged at 10,000 rpm for 1 minute and then the filtrate was discarded. The remaining cDNAs were also placed on the column and centrifuged in the same manner as described above. The column was transferred to a new 2 mL tube. 750 µl of cDNA Wash Buffer was placed on the column and centrifuged and then the filtrate was discarded. Further, the column was centrifuged at 14000 rpm for 5 minutes. The column was transferred to a new 1.5-mL tube, 14 µl of cDNA Elution Buffer was placed on the column and allowed to stand for 1 minute, followed by centrifugation at 14000 rpm for 1 minute to clean cDNA.

### (2-4) IVT labeling

Biotin-labeled cRNA was synthesized from the obtained cDNA by the in vitro transcription (IVT) reaction according to the following procedures. The following reagents were charged to a PCR tube, which was lightly mixed and incubated at 37°C for 16 hours, and then cRNA was obtained. The used reagents are included in a One-Cycle Target Labeling and Control Reagents Kit.

| |
|---|
| cDNA 12 µl from process (2-3) |
| RNase-free water 8 µl |
| 10 × IVT Labeling Buffer 4 µl |
| IVT Labeling NTP Mix 12 µl |
| IVT Labeling Enzyme Mix 4 µl |
| Total 40 µl |

### (2-5) Cleaning of cRNA

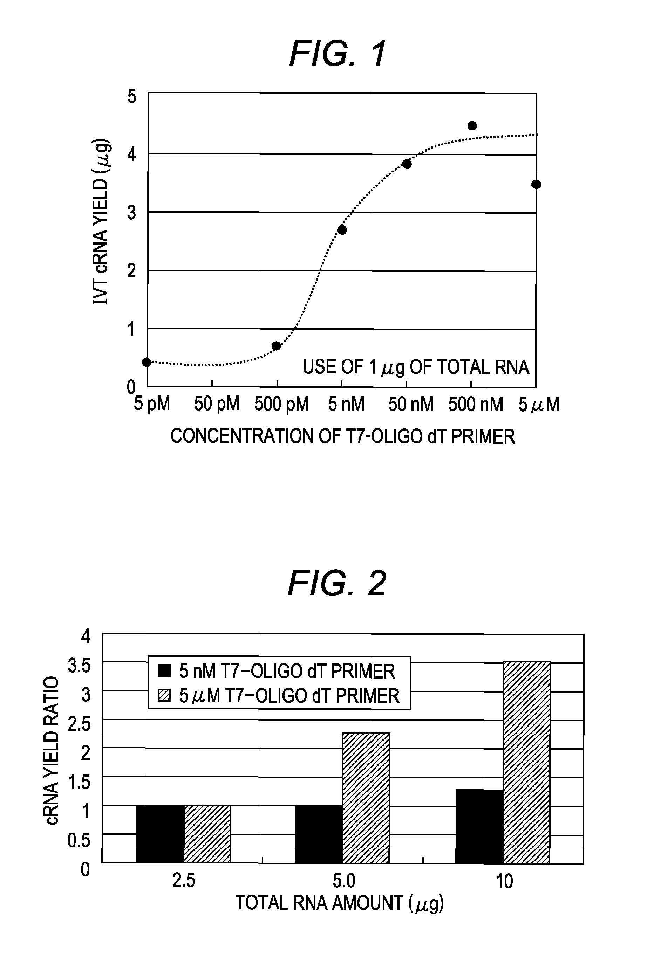

The obtained cRNA was transferred to a 1.5 mL tube and 60 µl of RNase-free water was added thereto, which was mixed with the vortex. 350 µl of IVT CRNA Binding Buffer was added thereto, which was mixed with the vortex. 250 µl of 100% EtOH was added to the mixture, which was mixed by pipetting. The resultant mixture was placed on cRNA Cleanup Spin Column and centrifuged at 1000 rpm for 15 seconds, followed by transferring of the column to a new tube. 500 µl of IVT cDNA Wash Buffer was charged to the column and centrifuged at 10000 rpm for 15 seconds and then the filtrate was discarded. 500 µl of 80% EtOH was charged thereto and centrifuged at 10000 rpm for 15 seconds and then the filtrate was discarded. After drying of the column by centrifugation at 14000 for 5 minutes, the column was transferred to a new tube, 11 µl of RNase-free water was placed on the column and allowed to stand for 1 minute, followed by centrifugation at 14000 rpm for 1 minute. Further, 10 µl of RNase-free water was placed on the column and allowed to stand for 1 minute, followed by centrifugation at 14000 rpm for 1 minute. The obtained filtrate was 200-fold diluted and the absorbance was measured. Then, the amount of cRNA was measured. The relationship between the concentration of each primer and the cRNA yield was shown in Fig. 1.

In order to maintain a constant yield of cRNA by adjusting the final concentration of T7-oligo dT primer, a condition in which the primer concentration is rate-limiting, namely, a state in which the amount of T7-oligo dT primer is low with respect to that of mRNA needs to be determined. The amount of cRNA to be obtained under the condition needs to be a sufficient amount for the nucleic acid detection with a microarray.

From Fig. 1, it is found that the suitable final concentration of T7-oligo dT primer is from 500 pM to 500 nM. In the following tests, the final concentration of the primer was set to 5 nM.

### Test example 2: Examination of relationship between amount of template RNA, and cRNA yield

A single-stranded cDNA was synthesized in the same manner as described in Test example 1 (2-1) by using 2.5, 5.0, or 10 µg of RNA obtained in the same manner as described in Test example 1 (1) as a template and using T7-oligo dT primer at a final concentration of 5 nM or 5 µm. Then, biotin-labeled cRNA was synthesized from the obtained single-stranded cDNA in the same manner as described in Test examples 1 (2-2) to (2-5) and the amount was measured. The results are shown in Table 1 below. The ratio of cRNA yield in the case where the cRNA yield was set to 1 when 2.5 µg of RNA was used as a template was shown in Fig. 2.

**(Table 1)**

| T7-oligo dT Final concentration of primer | Total RNA amount (µg) | cRNA yield (µg) |
|---|---|---|
| 5nM | 2.5 | 19.7 |
| | 5.0 | 19.6 |
| | 10 | 25.1 |
| 5µM | 2.5 | 53.3 |
| | 5.0 | 120.9 |
| | 10 | 188.3 |

As shown in Tables 1 and 2, in the case of the reaction system with a primer concentration of 5 µM, the amount of cRNA being obtained was increased in proportion to the amount of RNA used as a template. On the other hand, in the case of the reaction system with a primer concentration of 5 nM, a nearly constant amount of cRNA could be obtained independent of the amount of RNA used as a template.

Therefore, it is shown that a nearly constant amount of cRNA can be synthesized, without depending on the amount of template RNA, by the method of synthesizing a single-stranded cDNA and the method of preparing a sample in the present invention.

### Test example 3: Examination of relationship between amount of template RNA and measurement result of microarray

A single-stranded cDNA was synthesized in the same manner as described in Test example 1 (2-1) by using 1.0 or 10 µg of RNA obtained in the same manner as described in Test example 1 (1) as a template and using T7-oligo dT primer at a final concentration of 5 nM. Then, biotin-labeled cRNA was synthesized from the obtained single-stranded cDNA in the same manner as described in Test examples 1 (2-2) to (2-5) and the amount was measured. The obtained cRNA and the following reagents are charged to a tube, which was mixed and incubated at 94°C for 35 minutes. Then, the fragmented cRNA was stored at 4°C. The used reagents are included in the One-Cycle Target Labeling and Control Reagents Kit.

| |
|---|
| cRNA 10 µl |
| 5 × Fragmentation Buffer 8 µl |
| RNase-free water 22 µl |
| Total 40 µl |

The amount of gene expression was measured by hybridization with the GeneChip (registered trademark) using the resulting fragmented biotin-labeled cRNA. The used nucleic acid chip was Human Genome U133 Plus 2.0 Array. The hybridization conditions were as follows.

### <Hybridization solution>

Fragmented cRNA 15 µg

Control Oligo B2 5 µl

20 × Eukaryotic Hyb control 15 µl

2 × Hybridization Mix 150 µl

DMSO 30µl

Nuclease-free water up to 300 µl in total

### <Hybridization temperature conditions>

99°C, 5 minutes → 45°C, 5 minutes → 14000 rpm, 5 minutes

The staining and cleaning of the chip were performed using Fluidic Station 450 (Affymetrix) and GeneChip Hybridization Wash and Stain kit (Affymetrix) for staining hybridized target cRNA with a streptoavidin phycoerythrin conjugate in accordance with instructions for use. The scanning was performed using GeneChip Scanner 3000 (Affymetrix).

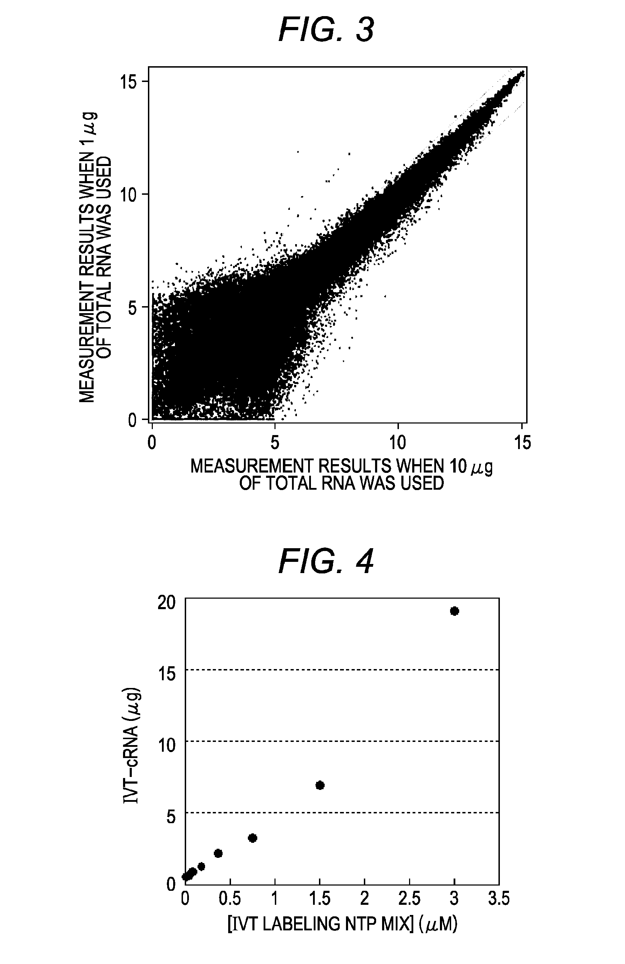

The image data scanned using a DNA microarray analysis software; GeneChip Operating Software (GCOS; Affymetrix) was converted to a CEL file, which was normalized with ArrayAssist (Stratagene) software. The correlation coefficient between the measurement results in each template RNA amount was calculated. The used algorithm for normalization was MAS5.0. The results are shown in Table 2 and Fig. 3. In Table 2, "SF" represents a coefficient multiplied when normalizing, "%P" represents a percentage of genes determined to be significantly expressed among genes which are detected with probes on the DNA chip, "%A" represents a percentage of genes determined to be unexpressed among genes which are detected with probes on the DNA chip, and "GAPDH3/5 ratio" represents a ratio of signal values of probes which detect the 3'side of a sequence of the GAPDH gene to signal values of probes which detect the 5' side (when the used RNA is degraded, a high value is shown; a standard is not more than 3).

**(Table 2)**

| Total RNA amount (µg) | cRNA yield (µg) | SF | Background average | Noise average | %P | %A | GAPDH 3/5 ratio |
|---|---|---|---|---|---|---|---|
| 1.0 | 27.87 | 2.71 | 45.21 | 2.52 | 40.91 | 57.89 | 1.17 |
| 10 | 38.18 | 1.99 | 52.36 | 2.88 | 44.41 | 54.45 | 0.95 |

The correlation coefficient between data regarding 1.0 µg and 10 µg of template RNA amount was as high as R² = 0.989. Therefore, in the method of detecting a nucleic acid of the present invention, it was suggested that a target nucleic acid could be detected with a microarray to the same extent despite the amount of RNA used as a template.

### Comparative example: Examination of relationship between concentration of NTP mix and cRNA yield in IVT reaction

In the method of synthesizing a single-stranded cDNA and the method of preparing a sample in the present invention, the objective for producing a constant amount of cRNA by adjusting the concentration of the primer to be used was solved. On the other hand, the present inventors examined a relationship between the NTP mix to be used as a substrate for RNA synthesis and the cRNA yield, in addition to the primer concentration. Namely, tests were performed in order to examine whether it was possible that the NTP mix to be used for the IVT reaction was depleted when a certain constant amount of product (cRNA) was obtained and the synthetic reaction was terminated.

A single-stranded cDNA was synthesized in the same manner as described in Test example 1 (2-1) by using 0.25 µg of RNA obtained in the same manner as described in Test example 1 (1) as a template and using T7-oligo dT primer at a final concentration of 5 µm. Then, biotin-labeled cRNA was synthesized from the obtained single-stranded cDNA in the same manner as described in Test examples 1 (2-2) to (2-5) except that the final concentration of IVT Labeling NTP Mix was set to 23.0 µM to 3.0 mM, and the amount was measured. The relationship between each NTP mix concentration and cRNA yield was shown in Fig. 4.

It was found that there was a proportionate relationship between the concentration of the NTP mix and the cRNA yield. Therefore, it was suggested that the NTP mix was not in a saturated state. The reason for this is assumed to be that the concentration of the NTP mix is rate-limiting for the reaction rate (the reaction rate is dependent on the concentration of the NTP mix).

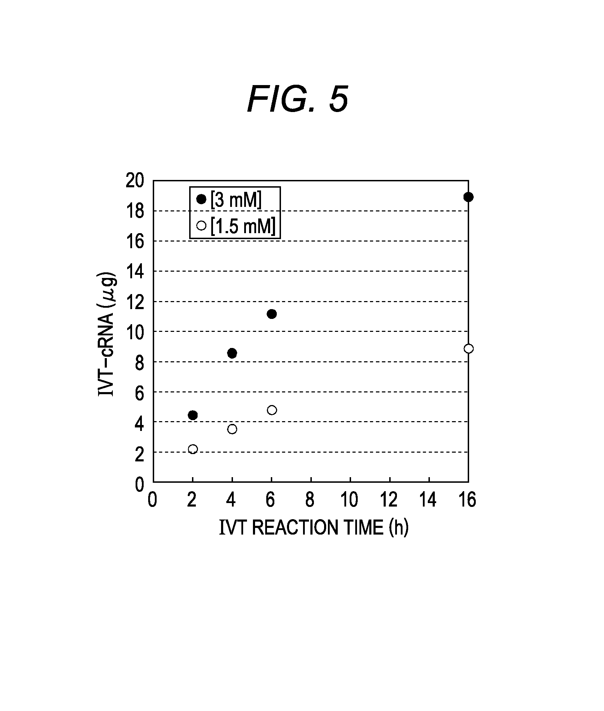

In order to confirm this point, a relationship between the reaction time (2 hours, 4 hours, 6 hours, 16 hours) and the cRNA yield when the NTP mix at a final concentration of 3.0 mM and 1.5 mM was used, was examined. The results are shown in Fig. 5.

From Fig. 5, it is found that the reaction rate changes depending on the concentration of the NTP mix. Therefore, it is expected that even if the concentration of NTP mix is decreased, the reaction rate is reduced, and thus the NTP mix is not depleted during the reaction. Accordingly, it is considered that a method of producing a constant amount of cRNA by adjusting the concentration of the NTP mix is impossible.

## Claims

1. A method of synthesizing a single-stranded cDNA, comprising:
reacting to synthesize a single-stranded cDNA using RNA extracted from the biological sample as a template and using a primer with oligo dT and a promoter sequence;
wherein the primer is used at a final concentration of 500 pM to 500 nM in the reaction for synthesizing a single-stranded cDNA.

2. The synthesizing method according to claim 1, wherein the biological sample is blood, lymph, urine, tissue, or cell samples.

3. The synthesizing method according to claim 1 or 2, wherein the concentration of RNA to be used as a template which is included in a reaction solution in the reaction for synthesizing a single-stranded cDNA is from 0.005 to 0.5 µg/µL.

4. The synthesizing method according to claim 1 to 3, wherein the primer is used at a final concentration of less than 200 nM, more particularly, less than 100 nm, less than 50 nm, less than 10 nm or 5n or less in the reaction for synthesizing a single-stranded cDNA.

5. A method of preparing a microarray sample, comprising:
extracting RNA from a biological sample;
synthesizing a single-stranded cDNA using a reaction solution containing the extracted RNA and a primer with oligo dT and a promoter sequence; and
synthesizing a target nucleic acid using the synthesized cDNA as a template to prepare a microarray sample;
wherein the final concentration of the primer in the reaction solution in the process of synthesizing a single-stranded cDNA is from 500 pM to 500 nM.

6. The method according to claim 4, wherein the biological sample is blood, lymph, urine, tissue, or cell samples.

7. The method according to claim 4 or 5, wherein the concentration of RNA to be used as a template in the process of synthesizing a single-stranded cDNA is from 0.005 to 0.5 µg/µL.

8. The method according to claim 5 to 7, wherein final concentration of the primer in the reaction solution in the process of synthesizing a single-stranded cDNA is less than 200 nM, more particularly, less than 100 nm, less than 50 nm, less than 10 nm or 5n or less.

9. The method according to any one of claims 5 to 8, wherein the target nucleic acid in the process of preparing a microarray sample is cRNA.

10. A method of detecting a nucleic acid with a microarray, comprising:
extracting RNA from a biological sample;
synthesizing a single-stranded cDNA using a reaction solution containing the extracted RNA and a primer with oligo dT and a promoter sequence;
synthesizing a target nucleic acid using the synthesized cDNA as a template to prepare a microarray sample;
bringing the prepared microarray sample into contact with a microarray and hybridizing the target nucleic acid included in the microarray sample with probes arranged on the microarray;
measuring a signal generated by hybridization of the target nucleic acid and the probe; and
detecting the target nucleic acid from the microarray sample based on the measurement results; wherein the final concentration of the primer in a reaction solution for synthesizing a single-stranded cDNA in the process of synthesizing a single-stranded cDNA is from 500 pM to 500 nM.

11. The method according to claim 8, wherein the biological sample is blood, lymph, urine, tissue, or cell samples.

12. The method according to claim 10 or 11, wherein the concentration of RNA to be used as a template in the process of synthesizing a single-stranded cDNA is from 0.005 to 0.5 µg/µL.

13. The method according to claim 10 to 12, wherein the final concentration of the primer in a reaction solution for synthesizing a single-stranded cDNA in the process of synthesizing a single-stranded cDNA is less than 200 nM, more particularly, less than 100 nm, less than 50 nm, less than 10 nm or 5n or less.

14. The method according to any one of claims 10 to 13, wherein the target nucleic acid in the process of preparing a microarray sample is cRNA.


SEQUENCE LISTING

<110>  Sysmex Corporation

<120>  A  method of synthesizing first strand cDNA and a method of preparing
 samples for microarray

<130>  SYSM-059-EP

<160>  1

<170>  PatentIn version 3.5

<210>  1
<211>  63
<212>  DNA
<213>  Artificial Sequence

<220>
<223>  primer

<400>  1
ggccagtgaa ttgtaatacg actcactata gggaggcggt tttttttttt tttttttttt       60

ttt                                                                     63